# EUROPEAN PATENT APPLICATION

(11) **EP 2 128 145 A2**
(43) Date of publication of application: **02.12.2009**
(21) Application number: 09006792.7
(22) Date of filing: 02.08.2007
(51) Int. Cl.: C07D 253/06, A61K 31/53

(54) **Improved method for synthesizing lamotrigine**

(30) Priority: 02.08.2006 US 834821 P
(62) Divisional of application: 07870418.6
(71) Applicant: Medichem, S.A., 08970 Sant Joan Despi, Barcelona (ES)
(72) Inventor: Arnalot Aguilar, Carmen, 17003 Girona Barcelona (ES)
(74) Representative: Duncan, Garreth Andrew

(57) **Abstract**

The invention relates, in general, to an improved process for preparing lamotrigine 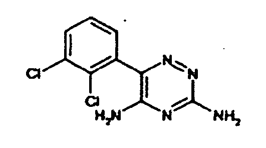 and related compounds. Processes for preparing and purifying lamotrigine, including lamotrigine hydrate, lamotrigine monohydrate and anhydrous lamotrigine, are described.

## Description

### CROSS REFERENCE TO RELATED APPLICATIONS

This application claims priority to United States Provisional Application No. 60/834,821, filed August 2, 2006, which is expressly incorporated herein by reference in its entirety.

### BACKGROUND OF THE INVENTION

### 1. Field of the Invention

The invention relates, in general, to an improved process for preparing lamotrigine.

### 2. Relevant Background

Lamotrigine is the common name for 3,5-diamino-6-(2,3-dichlorophenyl)-1,2,4-triazine which is a commercially marketed, pharmaceutically active substance known to be useful for the treatment of epilepsy and bipolar disorder. Lamotrigine (Formula I. below) has an empirical formula of C₉H₇N₅Cl₂ and a molecular weight of 256.09.

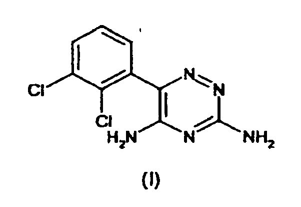

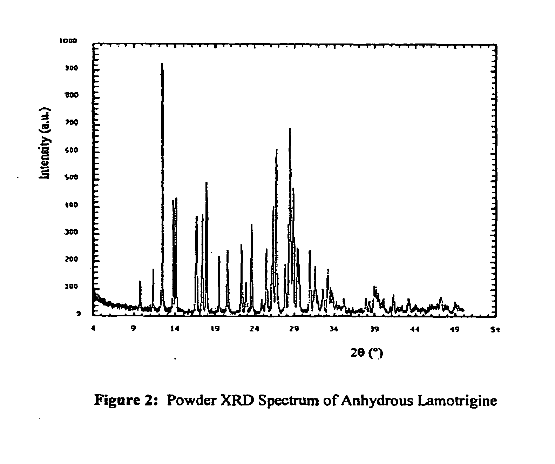

Lamotrigine is currently available in its anhydrous crystalline form which is characterized by the X-ray Powder Diffraction (XRD) spectrum shown in Figure 2.

Lamotrigine and its pharmaceutically acceptable acid addition salts are generally described in U.S. Patent No. 4,560,687 ("the '687 patent"). The '687 patent, however, provides no examples for preparing lamotrigine.

U.S. Patent No. 4,602,017 ("the `017 patent") discloses a process for preparing lamotrigine by cyclizing the intermediate 2-(2,3-dichlorophenyl)-2-guanidinylimino acetonitrile (Formula VI, below) by refluxing it in an alkanol in the presence of a strong base. The product is then treated with ice water, stirred for 30 minutes, filtered, and recrystallized to produce a residue, which is treated with isopropanol to yield lamotrigine. The process described in the '017 patent for preparing lamotrigine is illustrated in Scheme 1.

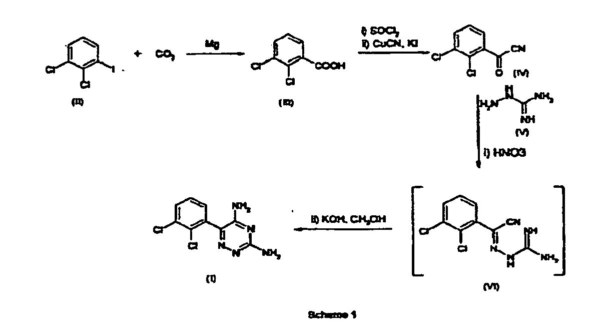

Various references disclose other different processes for producing lamotrigine. For example, WO 00/35888 discloses a process for preparing the intermediate of Formula IV, and describes preparing lamotrigine using this intermediate by a process similar to the one shown in Scheme 1 but using, in the last step, an aqueous solution of potassium hydroxide instead of the methanolic solution.

U.S. Patent No. 5,912,345 discloses cyclizing the intermediate of Formula VI using ultraviolet or visible radiation and heating to reflux temperature.

WO 01/49669 discloses the reaction of intermediates of Formula IV and Formula V to give intermediate Formula VI using sulfuric acid and p-toluenesulfonic acid. Cyclization is then performed by refluxing in an aliphatic alkanol in the presence of a base. The obtained lamotrigine is then purified by recrystallization or chromatographic separation to produce lamotrigine with a purity of 99.70 (calculated by HPLC).

U.S. Patent No. 5,925,755 discloses preparing lamotrigine from 6-(2,3-dichlorophenyl)-5-chloro-3-thiomethyl-1,2,4-triazine that has been dissolved in ethanol saturated with ammonia gas, by heating in a sealed glass tube in an autoclave at 180° C/1930 kPa for 72 hours followed by recrystallizing from methanol.

Lamotrigine is known to form solvates with different alcohols. For example, according to an article published in 1989 by Robert W. Janes et al- in Acta Cryst. (1989), C45, 129-132, entitled "Structure of Lamotrigine Methanol Solvate: 3,5-Diamino-6-(2,3-dichlorophenyl)-1,2,4-triazine-Methanol, A Novel Anticonvulsant Drug," the crystal structure of a sample of "lamotrigine methanol solvate" was provided by the Wellcome Research Laboratories, UK, and a second form of lamotrigine was crystallized from absolute ethanol. According to EP 0 800 520 B 1, published on October 15, 1997, "lamotrigine ethanolate" can be obtained by crystallization of lamotrigine from ethanol (see Examples 5 and 6). Similarly, EP 0 021 121 teaches that "lamotrigine isoproponate" can be obtained by recrystallization from isopropanol *(see* Example 1). Lamotrigine alcohol solvates can be dried to yield anhydrous lamotrigine. For example, WO 96/20935 describes the drying and recystallization from methanol of anhydrous lamotrigine having a meriting point of 218° C (see Example 6).

Most of the known processes for preparing lamotrigine do so only in low yields and by using drastic conditions (*e.g*., temperature and pressure) or by using dangerous reagents or expensive equipment. Moreover, the known processes only describe preparing lamotrigine as a crude product that is then purified by recrystallization from a solvent and fail to disclose any other processes for purifying lamotrigine.

It is necessary to prepare pharmaceutical products, such as lamotrigine, of a high grade and having minimum amounts of impurities. In particular, the presence of impurities in the product may adversely effect safety as well as negatively affect formulation shelf life. As such, there is a need for simplified processes for producing lamotrigine having a high purity in high yields.

### SUMMARY OF THE INVENTION

The invention provides an improved method for producing lamotrigine that includes preparing lamotrigine by the cyclization of 2-(2,3-dichlorophenyl)-2-guanidinylimino acetonitrile (Formula VI).

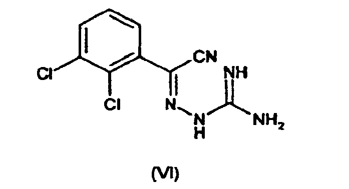

The process further includes addition of methanesulfonic acid to the crude lamotrigine, obtaining a solution of lamotrigine methanesulfonate, and filtering the insoluble or mechanical particles from the solution of lamotrigine methanesulfonate. Preferably, methanesulfonic acid is added to the crude lamotrigine until a pH of approximately 1 to approximately 2 is obtained.

The process further includes readjusting the pH to approximately 6.5 to approximately 7.5, preferably to approximately 6.8 to approximately 7.2, stirring the mixture for approximately 1 hour, filtering the precipitated Lamotrigine hydrate, washing the precipitated lamotrigine hydrate with water and crystallizing the obtained residue with an alcohol (*e.g*., methanol).

One aspect of the invention includes a process for the cyclization of 2-(2,3-dichlorophenyl)-2-guanidinyliminoacetonitrile without the need to use a base.

Another aspect of the invention includes a process for purifying lamotrigine to obtain lamotrigine substantially free of insoluble or mechanical particles. Lamotrigine, and most of its acid addition salts (*e.g*., acetate, succinate, maleate, citrate, hydrochloride), are very insoluble materials. As such, the removal of insoluble or mechanical impurities from the product can be difficult to accomplish. This difficulty is alleviated in the invention via preparing a solution of the lamotrigine methanesulfonate salt.

Another aspect of the invention includes a new form of lamotrigine, crystalline lamotrigine hydrate, in particular lamotrigine monohydrate, as well as a method for preparing them. Lamotrigine monohydrate is characterized herein by its XRD spectrum.

The invention further includes the use of the novel crystalline lamotrigine hydrate (the synthesis of which provides an extra purification step) to produce anhydrous lamotrigine. Thus, the invention further includes a process for producing essentially anhydrous lamotrigine. The anhydrous lamotrigine obtained according to the process of the invention is substantially free of insoluble or mechanical particles and has a purity of at least approximately 99% as measured by HPLC.

### BRIEF DESCRIPTION OF THE DRAWING

The accompanying drawings, which are included to provide a further understanding of the invention and are incorporated in and constitute a part of this specification, illustrate embodiments of the invention and together with the description serve to explain the principles of the invention. In the drawings:
Figure 1 illustrates the powder XRD spectrum of lamotrigine monohydrate; and
Figure 2 illustrates the powder XRD spectrum of anhydrous lamotrigine.

### DETAILED DESCRIPTION OF THE PREFERRED EMBODIMENTS

Reference will now be made in detail to the preferred embodiments of the invention. This invention may, however, be embodied in many different forms and should not be construed as limited to the embodiments set forth herein. In addition, and as will be appreciated by one of skill in the art, the invention may be embodied as a method, system or process.

One aspect of the invention includes a process for the cyclization of 2-(2,3-dichlorophenyl)-2-guanidinyliminoacetonitrile in which the process only requires refluxing Compound VI in an alcohol. Preferably the alcohol is isopropanol, but other alcohols, including ethanol, butanol and combinations thereof may also be used. Cyclization of 2-(2,3-ichlorophenyl)-2-guanidinyliminoacetonitrile can be accomplished by combining an alcohol, preferably isopropanol, with 2-(2,3-dichlorophenyl)-2-guanidinyliminoacetonitrile followed by heating at reflux temperature. The mixture is then maintained at reflux temperature for approximately 6 hours. The mixture is then cooled to approximately 20 to approximately 25° C, kept at this temperature, while stirring, for approximately 1 hour, filtered and washed with an alcohol (*e.g*., isopropanol).

Another aspect of the invention includes a process for purifying lamotrigine by removing insoluble and/or mechanical impurities of lamotrigine comprising:
i. combining water and lamotrigine;
ii. adjusting the pH of the mixture between approximately I to approximately 2 by the addition of methanesulfonic acid while keeping the temperature below approximately 40° C to obtain an aqueous solution of lamotrigine methanesulfonate;
iii. filtering the insoluble and/or mechanical particles from the solution;
iv. adjusting the pH of the filtrate to between approximately 6.5 to approximately 7.5, preferably between approximately 6.8 to approximately 7.2, by the addition of 50% aqueous solution of sodium hydroxide; and
v. filtering the precipitate.

The above described process can further optionally include a step (vi) drying the obtained solid; a step (vii) crystallizing the obtained solid, if desired; and/or a step (viii) drying the obtained solid. Notably, steps (vii) and (viii) are needed for obtaining anhydrous lamotrigine.

The above described process can further include adding a decolorizing agent to the aqueous solution of lamotrigine methanesulfonate obtained in step (ii) before performing step (iii) in order to improve the color and purity of the resulting lamotrigine crystals. The decolorizing agent can be any conventional decolorizing agent, including, but not limited to, alumina, activated alumina, silica and charcoal.

The above described process yields lamotrigine hydrate.

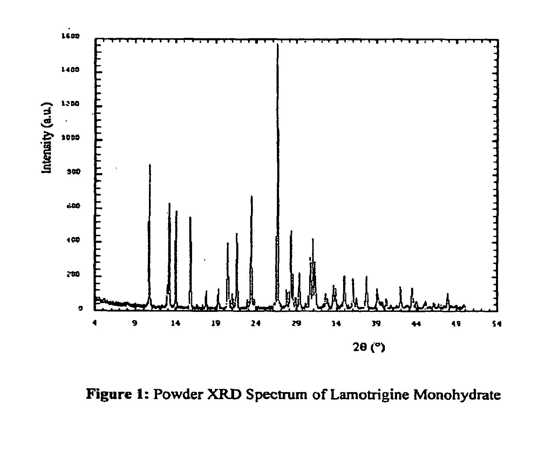

Another aspect of the invention includes a form of lamotrigine monohydrate characterized by the powder XRD spectrum illustrated in Figure 1.

Another aspect of the invention includes a form of lamotrigine monohydrate characterized by a powder XRD spectrum (2θ) ± 0.2°) having the following peaks at approximately: 10.7°, 13.2°, 14.0°, 15.8°, 20.5°, 21.5°, 23.4°, 26.5°, 28.2°, 28-5°, 29.3°, 30.6°, 30.9°, 31.2°, 34.9° and 37.7°.

Another aspect of the invention includes a process for preparing lamotrigine monohydrate characterized by the powder XRD spectrum illustrated in Figure 1, wherein the process includes removing insoluble or mechanical particles after forming an aqueous solution of methanesulfonate salt of lamotrigine, optionally decoloring the solution, filtering the solution, neutralizing the filtrate and drying the precipitate.

Another aspect of the invention includes the use of the crystalline lamotrigine hydrate (the synthesis of which provides an extra purification step) to produce the anhydrous lamotrigine.

Another aspect of the invention includes a process for producing substantially anhydrous lamotrigine comprising:
i. combining lamotrigine hydrate and methanol;
ii. heating the mixture at reflux temperature;
iii. cooling the mixture to approximately 20 to approximately 25° C and maintaining the mixture at this temperature, while stirring, for approximately 1 hour resulting in a slurry;
iv. centrifuging the slurry from step (iii) to obtain wet lamotrigine methanolate; and
v. drying the wet lamotrigine methanolate to yield anhydrous lamotrigine.

The anhydrous lamotrigine obtained according to any of the processes of the invention is substantially free of insoluble or mechanical particles and has a purity of at least approximately 99%, preferably of at least approximately 99.5%, and more preferably of at least approximately 99.9%, when analyzed by HPLC.

The invention further includes formulating lamotrigine prepared by the above described processes into readily usable dosage units for the therapeutic treatment (including prophylactic treatment) of mammals including humans. Such formulations are normally formulated in accordance with standard pharmaceutical practice as a pharmaceutical composition. According to this aspect of the invention there is provided a pharmaceutical composition that comprises lamotrigine prepared by the above described processes as defined hereinbefore in association with a pharmaceutically acceptable diluent or carrier.

The compositions of the invention may be in a form suitable for oral use (for example as tablets, fast-dissolving tablets, lozenges, hard or soft capsules, aqueous or oily suspensions, emulsions, dispersible powders or granules, syrups or elixirs). For example, compositions intended for oral use may contain, for example, one or more coloring, sweetening, flavoring and/or preservative agents.

Suitable pharmaceutically-acceptable excipients for a tablet formulation include, for example, inert diluents such as lactose, sodium carbonate, calcium phosphate, calcium carbonate and different types of cellulose such as powdered cellulose or microcrystalline cellulose; granulating and disintegrating agents such as corn starch and its derivatives, crosspovidone, crosscarmellose and/or algenic acid; binding agents such as starch and ' pregelatinized starch; lubricating agents such as magnesium stearate, stearic acid or talc; preservative agents such as sodium benzoate, ethyl or propyl p-hydroxybenzoate; and anti-oxidants, such as ascorbic acid. Tablet formulations may be uncoated or coated either to modify their disintegration and the subsequent absorption of the active ingredient within the gastrointestinal tract, or to improve their stability and/or appearance, in either case, using conventional coating agents and procedures well known in the art.

Compositions for oral use may be in the form of hard gelatin capsules in which the active ingredient is mixed with an inert solid diluent, for example, calcium carbonate, calcium phosphate, kaolin or cellulose, a disintegrating agent such as corn starch and its derivatives, crosspovidone and crosscarmellose, or as soft gelatin capsules in which the active ingredient is mixed with water or an oil such as peanut oil, liquid paraffin, olive oil or glyceryl oleate derivatives.

Aqueous suspensions generally contain the active ingredient in finely powdered form together with one or more suspending agents, such as sodium carboxymethylcellulose, methylcellulose, hydroxypropylmethylcellulose, sodium alginate, polyvinyl-pyrrolidone, gum tragacanth and gum acacia; dispersing or wetting agents such as lecithin or condensation products of an alkylene oxide with fatty acids (for example polyoxethylene stearate), or condensation products of ethylene oxide with long chain aliphatic alcohols, for examples heptadecaethyleneoxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives (such as the sodium salt of benzoic acid, ethyl or propyl p-hydroxybenzoate), anti-oxidants (such as ascorbic acid), coloring agents, flavoring agents, and/or sweetening agents (such as sucrose, saccharine or aspartame).

Oily suspensions may be formulated by suspending the active ingredient in a vegetable oil (such as arachis oil, olive oil, sesame oil or coconut oil) or in a mineral oil (such as liquid paraffin). The oily suspensions may also contain a thickening agent such as beeswax, hard paraffin or cetyl alcohol. Sweetening agents such as those set out above, and flavoring agents may be added to provide a palatable oral preparation. These compositions may be preserved by the addition of an anti-oxidant such as ascorbic acid.

Dispersible powders and granules suitable for preparation of an aqueous suspension by the addition of water generally contain the active ingredient together with a dispersing or wetting agent, suspending agent and one or more preservatives. Suitable dispersing or wetting agents and suspending agents are exemplified by those already mentioned above. Additional excipients such as sweetening, flavoring and coloring agents, may also be present.

The pharmaceutical compositions of the invention may also be in the Form of oil-in-water emulsions. The oily phase may be a vegetable oil, such as olive oil or arachis oil, or a mineral oil, such as for example liquid paraffin or a mixture of any of these. Suitable emulsifying agents may be, for example, naturally-occurring gums such as gum acacia or gum tragacanth, naturally-occurring phosphatides such as soya bean, lecithin, an esters or partial esters derived from fatty acids and hexitol anhydrides (for example sorbitan monooleate) and condensation products of the said partial esters with ethylene oxide such as polyoxyethylene sorbitan monooleate. The emulsions may also contain sweetening, flavoring and preservative agents.

Syrups and elixirs may be formulated with sweetening agents such as glycerol, propylene glycol, sorbitol, aspartame or sucrose, and may also contain a demulcent, preservative, flavoring and/or coloring agent.

The amount of a compound of this invention that is combined with one or more excipients to produce a single dosage form will necessarily vary depending upon the host treated and the particular route of administration. For example, a formulation intended for oral administration to humans may contain, for example, from 0.5 mg to 2 g of active agent compounded with an appropriate and convenient amount of excipients which may vary from about 5 to about 98 percent by weight of the total composition.

The size of the dose for therapeutic or prophylactic purposes of the compounds of the invention will naturally vary according to the nature and severity of the conditions, the age and sex of the animal or patient and the route of administration, according to well known principles of medicine. For example, the method may comprise at least one of an hourly administration, a daily administration, a weekly administration, or a monthly administration of one or more compositions described herein.

According to the present invention, suitable methods of administering the therapeutic composition of the present invention to a patient include any route *of in* vivo administration that is suitable for delivering the composition into a patient. The preferred routes of administration will be apparent to those of skill in the art, depending on the type of condition to be prevented or treated, and/or the target cell population.

It will be apparent to those skilled in the art that various modifications and variations can be made in the present invention and specific examples provided herein without departing from the spirit or scope of the invention. Thus, it is intended that the present invention covers the modifications and variations of this invention that come within the scope of any claims and their equivalents.

### EXAMPLES

The following examples are for illustrative purposes only and are not intended, nor should they be interpreted to, limit the scope of the invention.

### General Experimental Conditions:

### I. HPLC Assay Method:

The chromatographic separation was carried out at room temperature (20-25° C) using a Lichrosphere RP-select B, 5 µm,4.0 x 250 mm I.D. column.

The mobile phase was prepared by mixing 320 mL of acetonitrile with 680 mL of buffer (pH = 5.6) prepared from 3.85 g of ammonium acetate dissolved in 1000 mL of water and by adjusting the pH to 5.6 with glacial acetic acid. The mobile phase was mixed and filtered through a 0.22 µm nylon filter under vacuum.

The chromatograph was equipped with a 306 nm detector, and the flow rate was 1.0 mL per minute. Test samples (20 µL) were prepared by dissolving the appropriate amount of sample in order to obtain 1 mg/mL of acetonitrile.

### Example 1: Preparation of 3,5-diamino-6-(2,3-dichlorophenyl)-1,2,4-triazine

### Step 1. Preparation of Lamotrigine

In a 800 L reactor, 38 kg of 2-(2,3-dichlorophenyl)-2-guanidinyliminoacetonitrile (0.148 kmoles) and 298 kg of isopropanol were combined. The mixture was then heated to reflux temperature (approximately 82° C) and maintained at 82 ± 3° C for 6 hours. Thereafter, the mixture was cooled to 20-25° C and stirred for 1 hour at this temperature. The suspension was then filtered and washed with 8 kg of isopropanol.

### Step 2. Removal of Mechanical Impurities

The crude lamotrigine obtained in Step I and 335 kg of deionized water were combined in the 800 L reactor, and the temperature was adjusted to between 35 and 40° C. Methanesulfonic acid was then added to the mixture until the pH was between 1.5 and 2.0 while maintaining the temperature between 35 to 40° C. The resulting aqueous solution of lamotrigine methane sulfonate was then filtered, and the filter and reactor were washed with 4 kg of deionized water.

The solution of lamotrigine methanesulfonate was then added to the 800 L reactor, and the pH was adjusted to 6.5 to 7.5 by adding a 50% solution of sodium hydroxide. Next, the temperature was adjusted at 20 to 25° C, and the mixture was stirred at this temperature for 1 hour. Thereafter, the suspension was filtered and washed with 20 kg of deionized water followed by 8 kg of methanol, to obtain wet lamotrigine monohydrate.

### Step 3. Conversion of Lamotrigine Hydrate to Anhydrous Lamotrigine

In the 800 L reactor, the wet lamotrigine hydrate obtained in Step 2 and 255 kg of filtered methanol were combined, and the mixture was heated to reflux temperature. The mixture was then cooled to 20-25° C and stirred at this temperature for 1 hour. Thereafter, the suspension was filtered and washed with 8 kg of methanol.

The wet product obtained was dried under vacuum for 16 hours at 85 ± 5° C and then was milled and sieved (500 µm to yield 29.11 kg (0.114 kmoles) of lamotrigine (Yield: 76.8%; Purity (HPLC analysis): 99.9%; Melting Point = 216° C).

### Example 2: Preparation of Lamotrigine Monohydrate

Lamotrigine (46 g) and 460 mL of deionized water were combined, and the temperature of the mixture was adjusted at 3 5 to 40° C. Initially, the pH of the mixture was 4.11. Methanesulfonic acid was then added to the mixture until the pH was 1.4 while maintaining the temperature of the mixture at or below 30° C. A light, opaline solution was obtained following addition of the methanesulfonic acid. The obtained solution of lamotrigine methanesulfonate was then filtered and the filter was washed with 4.6 mL of deionized water.

The pH of the solution of lamotrigine methanesulfonate was next adjusted to 6.8-7.2 by adding a 50% solution of sodium hydroxide while maintaining the temperature at or below 30° C. The temperature was then adjusted to between 20° C and 25° C, and the mixture was stirred at this temperature for 1 hour. Thereafter, the suspension was filtered, and the obtained product was washed with deionized water and dried at 40° C to yield 49.23 g of lamotrigine monohydrate. Analysis: titration (perchloric acid): 99.23%; purity (HPLC analysis): 99%; Water (Karl Fischer) = 6.68%.

Although the invention has been described and illustrated with a certain degree of particularity, it is understood that the present disclosure has been made only by way of example, and that numerous changes in the conditions and order of steps can be resorted to by those skilled in the art without departing from the spirit and scope of the invention.

## Claims

1. Lamotrigine monohydrate, wherein said lamotrigine monohydrate is **characterized by** a powder XRD spectrum (2θ) (± 0.2°) having peaks at approximately 10.7°, 13.2°, 14.0°, 15.8°, 20.5°, 21.5°, 23.4°, 26.5°, 28.2°, 28.5°, 29.3°, 30.6°, 30.9°, 31.2°, 34.9° and 37.7°.

2. A process for preparing lamotrigine monohydrate, said process comprising:
i. preparing an aqueous solution of lamotrigine methanesulfonate;
ii. removing any insoluble or mechanical particles from said aqueous solution of step (i);
iii. neutralizing said aqueous solution of step (ii) to obtain an aqueous suspension;
iv. filtering said suspension obtained in step (iii); and
v. optionally, drying the resulting precipitate.

3. The process of claim 2, wherein said step of preparing an aqueous solution of lamotrigine methanesulfonate comprises:
i. combining lamotrigine and water to obtain a mixture; and
ii. adjusting the pH of said mixture to between approximately 1 and approximately 2 by the addition of methanesulfonic acid while keeping the temperature of said mixture below approximately 40° C.

4. The process of claim 2, wherein said step of neutralizing said aqueous solution of step (ii) to obtain an aqueous suspension comprises:
i. adjusting the pH of the filtrate to between approximately 6.5 to approximately 7.5 by adding an approximately 50% solution of aqueous sodium hydroxide; and
ii. stirring the filtrate for approximately 1 hour at approximately 25° C.

5. The process of claim 4, wherein said addition of an approximately 50% solution of sodium hydroxide results in a pH of approximately 6.8 to approximately 7.2.

6. The process of any of claims 2-5 further comprising decolorizing said aqueous solution of lamotrigine methanesulfonate by treating said solution with a decolorizing agent.

7. The process of claim 6, wherein said decolorizing agent is at least one of alumina, activated alumina, silica, charcoal and combinations thereof.

8. A process for purifying lamotrigine and related compounds, said process comprising at least one of the processes of claims 2-7.

9. A process for preparing anhydrous lamotrigine comprising converting said lamotrigine monohydrate of claim 1 into anhydrous lamotrigine.

10. The process of claim 9, further comprising:
i. combining lamotrigine monohydrate and methanol to form a mixture;
ii. heating the mixture of step (i) to reflux;
iii. cooling and stirring the mixture obtained in step (ii) to obtain a slurry;
iv. centrifuging said slurry; and
v. drying the obtained solid.

11. Anhydrous lamotrigine prepared according to any of claims 9 or 10, wherein said anhydrous lamotrigine is substantially free of insoluble or mechanical particles.

12. Anhydrous lamotrigine, wherein said anhydrous lamotrigine has a purity of at least approximately 99% as measured by HPLC.

13. Anhydrous lamotrigine, wherein said anhydrous lamotrigine has a purity of at least approximately 99.5% as measured by HPLC.

14. Anhydrous lamotrigine, wherein said anhydrous lamotrigine has a purity of at least approximately 99.9% as measured by HPLC.
